# FASCICULE DE BREVET EUROPEEN

(11) **EP 1 161 157 B1**
(45) Date de publication et mention de la délivrance du brevet: **04.08.2004**
(21) Numéro de dépôt: 00909391.5
(22) Date de dépôt: 13.03.2000
(51) Int. Cl.: A23L 1/30, A23L 1/307

(54) **COMPLEMENT ALIMENTAIRE ET PROCEDE DE TRAITEMENT COSMETIQUE A BASE D'UN EXTRAIT DE RAISIN RICHE EN POLYPHENOLS**
NAHRUNGSERGÄNZUNGSMITTEL UND VERFAHREN ZUR KOSMETISCHEN BEHANDLUNG UNTER VERWENDUNG VON EINEM POLYPHENOLREICHEN TRAUBENEXTRAKT
FOOD COMPLEMENT AND METHOD FOR COSMETIC TREATMENT BASED ON A GRAPE EXTRACT RICH IN POLYPHENOLS

(30) Priorité: 12.03.1999 FR 9903076
(43) Date de publication de la demande: 12.12.2001
(73) Titulaire: Laboratoires Arkopharma, 06510 Carros (FR)
(72) Inventeur: ROMBI, Max, I-18022 Bordighera (IT)
(74) Mandataire: Texier, Christian
(86) Numéro de dépôt international: PCT/FR2000/000485
(87) Numéro de publication internationale: WO 2000/054610

(56) Documents cités:
- WO-A-90/13304
- WO-A-99/01148
- FR-A- 2 659 556
- DATABASE WPI Section Ch, Week 199545 Derwent Publications Ltd., London, GB; Class D13, AN 1995-345078 XP002125460 & CN 1 094 901 A (WANG J), 16 novembre 1994 (1994-11-16)

## Description

La présente invention concerne le domaine général des compléments alimentaires à visées diététiques et/ou cosmétiques. Elle vise également un procédé de traitement cosmétique et en particulier une action contre la cellulite. L'invention concerne donc en premier lieu le domaine très général du traitement de l'obésité.

Les extraits de raisin riches en polyphénols sont connus par WO 90/13304 et WO 99/01148.

L'objectif thérapeutique en matière d'obésité est bien défini: il s'agit soit de permettre au sujet de perdre du poids de façon significative, soit d'aider le sujet à conserver un niveau de poids aussi bas que souhaitable.-

Plusieurs types d'approches ont été envisagés à ce jour.

Les approches nutritionnelles visent à réduire l'apport d'énergie sous forme d'aliments. Cela peut se faire en réduisant drastiquement les apports énergétiques ou en remplaçant des nutriments énergétiques par d'autres qui le sont moins: tels que les graisses non digestibles de substitution, les triglycérides structurés à assimilation réduite ou les fibres alimentaires non assimilables.

Les approches thérapeutiques peuvent avoir des cibles diverses.
- La réduction de la prise alimentaire peut être le premier objectif. La réduction de la prise alimentaire peut être recherchée par l'utilisation de substances anorexigènes, dont les effets à court terme sont montrés, mais dont la durée d'utilisation est limitée à cause d'effets secondaires indésirables. En fait, très peu de ces produits sont véritablement utilisables et leur efficacité à long terme reste très discutée. De nouvelles molécules sont en cours d'évaluation ou pourraient l'être dans un avenir proche, mais leur intérêt reste encore à démontrer.
- Un deuxième objectif peut être l'augmentation de la dépense énergétique par l'utilisation des substances thermogéniques agissant au niveau central ou périphérique. L'utilisation de ces substances reste encore limitée.
- Un troisième objectif est de réduire l'assimilation des lipides alimentaires, voire éventuellement celle des glucides. Il s'agit d'une approche plus récente mais qui connaît un intérêt grandissant. Une réduction de l'assimilation des lipides alimentaires peut être obtenue soit par une réduction de l'activité des enzymes digestives concernées, soit en modifiant les propriétés des interfaces transportant les molécules lipidiques, émulsions, vésicules ou micelles.

La présente invention concerne l'objet des revendications indépendantes 1, 2 et 9.

Selon une autre caractéristique l'extrait utilisé selon l'invention contient de 10 à 60% en poids de proanthocyanidols.

Selon une autre caractéristique l'extrait utilisé selon l'invention contient de 0,001 à 0,1% en poids de trans-resvératrol.

Les extraits de raisin, utilisés conformément à la présente invention, peuvent être obtenus soit à partir de marc de raisin, soit encore à partir de pépins et/ou d'enveloppes de grains et/ou éventuellement de rafles de raisin.

De façon générale, les extraits de raisin contiennent des polyphénols et notamment des proanthocyanidols et des anthocyanosides.

Les polyphénols extraits des raisins ont des activités biologiques multiples :
- les proanthocyanidols peuvent être considérés comme de puissants capteurs de radicaux libres qui freineraient l'oxydation des LDL responsables de la formation de plaques d'athéromes.
- L'action des oligomères procyanidoliques (OPC) sur les parois vasculaires, mise en évidence chez l'animal et confirmée chez l'homme, se traduit par une augmentation de la résistance des capillaires sanguins et par une - diminution de leur perméabilité.
- Les polyphénols préservent les protéines fibreuses, notamment le collagène et l'élastine contre la dégradation enzymatique.
- Les polyphénols entraînent également une baisse du taux de cholestérol dans le sang, et
- Ils présentent une activité anti-agrégante plaquettaire.

D'autres actions ont également été envisagées et en particulier une action des polyphénols en tant qu'agents anti-inflammatoires, protecteurs vasculaires, anticaries, antihistaminiques, anticarcinogéniques, ou protecteurs solaires.

Les dérivés polyphénoliques non flavonoïdes, parmi lesquels se distingue le resvératrol, seraient également dotés de qualités antioxydantes et pourraient jouer un rôle antiviral, anticarcinogène et immunorégulateur.

Dans le cadre de la présente invention, les extraits de raisin présents dans les compléments alimentaires, sont riches ou enrichis en polyphénols, pour leur conférer une activité d'inhibition des lipases digestives.

Une étude a permis de démontrer qu'un extrait de raisin conforme à la présente invention, à la dose de 6 mg pour 100 mg de lipides, permettait de supprimer totalement l'émulsification des lipides en milieu gastrique.

En revanche, dans le milieu duodénal, un tel extrait diminue de manière significative, de l'ordre de 16%, l'émulsification des lipides sans la supprimer totalement. Lorsque l'on sait que l'émulsification des lipides est l'étape indispensable à l'action des lipases sur les lipides alimentaires, ces résultats démontrent la capacité d'inhibition mécanique et donc réversible des lipases digestives, préférables à une inhibition chimique pouvant être irréversible.

Une autre étude in vitro, réalisée dans des conditions reproduisant les conditions physiologiques , c'est-à-dire action successive sur la trioléine de lipase gastriquqe puis de lipase pancréatique, a démontré que l'extrait de raisin selon l'invention, à la dose de 6 mg/100 mg de lipides, permettait une inhibition presque totale de la lipase gastrique ( 78 % d'inhibition ) et de la lipase pancréatique (52% d'inhibition), soit une inhibition de la lipolyse totale de près de 60%.

Dans le cadre de la présente invention, des travaux ont été conduits pour étudier l'effet de l'extrait de raisin sur la thermogenèse. Ces travaux ont été réalisés sur un modèle pharmacologique ex vivo, dont le principe est de mesurer la consommation d'oxygène d'un échantillon de tissu adipeux brun de rat ; la consommation d'oxygène est proportionnelle à la thermogenèse induite dans le tissu adipeux brun par l'extrait testé.

| **Concentration d'extrait de** **raisin dans le milieu** **(mg/100ml)** | **Consommation d'oxygène** **(millimole d'oxygène/mg)** |
|---|---|
| 0 | 43 |
| 20 | 90 |
| 40 | 136 |
| 60 | 156 |

On constate que cet extrait induit une augmentation importante (110 %) de la thermogenèse dès la plus faible concentration.

On indiquera ci-après à titre d'exemple un mode de réalisation particulier d'obtention d'un extrait de raisin susceptible d'être utilisé dans le cadre de la présente invention.

La matière de départ (marc et/ou pépins) contient de 0,1 à 5% d'OPC et de 0,0001 à 0,005% de trans-resvératrol.

Afin d'obtenir une inhibition des lipases avec une posologie raisonnable, il est nécessaire d'avoir recours à un extrait apportant, sous un faible volume, les doses nécessaires de polyphénols. A titre d'exemple, le -procédé d'extraction suivant peut être mis en oeuvre : 1 kg de marc (ou pépins) est extrait par 5 kg d'éthanol à 60% (V/V). Après filtration, l'extrait est concentré sous vide partiel à une température maximale de 80°C. Un extrait concentré est ensuite séché sous vide (température maximale de 80°C) ou par atomisation (à 200°C maximum) avec ou sans maltodextrine, en fonction des spécifications en traceurs retenues. L'extrait sec ainsi obtenu a une teneur en OPC comprise entre 10 et 40% d'OPC et entre 0,001 et 0,05 de trans-resvératrol selon la teneur de ces composants dans la matière première végétale.

Cet exemple de mise en oeuvre d'un procédé de tels extraits n'est pas limitatif, c'est ainsi qu'il est possible d'utiliser d'autres solvants, notamment le méthanol et éventuellement un antioxydant (l'acide ascorbique, le métabisulfite de sodium, etc) pour éviter l'oxydation des polyphénols.

Un tel extrait concentré peut le cas échéant faire l'objet d'une seconde extraction, notamment à l'acétate d'éthyle, pour obtenir un extrait sec ayant une teneur en OPC supérieure à 50%.

La teneur en OPC peut par exemple être déterminée par mise en oeuvre de la méthode analytique suivante. La matière première à analyser est extraite par un mélange eau-acétone (10-30 V/V). Après dilution, la solution extractive est chargée sur une cartouche contenant de la phase stationnaire C18 en phase inversée. Après rinçage, les OPC sont élués à l'acétate d'éthyle. La solution purifiée est dosée par colorimétrie avec le réactif à la vanilline sulfurique contre témoin de catéchine.

L'invention se rapporte également à l'objet de la revendication 4.

## Revendications

1. Utilisation d'un extrait de raisin, riche ou enrichi en polyphénols, à raison de 30 à 90 % en poids de polyphénols, destiné à une administration par voie orale, pour traiter la cellulite et/ou la surcharge pondérale.

2. Utilisation d'un extrait de raisin, riche ou enrichi en polyphénols, à raison de 30 à 90 % en poids de polyphénols, pour la fabrication d'un médicament, destiné à une administration par voie orale, pour traiter l'obésité.

3. Utilisation selon la revendication 1, **caractérisée en ce qu'**elle implique l'administration d'une dose journalière de 0,2 à 2 grammes dudit extrait de raisin.

4. Utilisation selon la revendication 2, **caractérisée en ce que** le médicament est conditionné sous une forme de dosage unitaire destinée à une dose d'utilisation journalière de 0,2 à 2 grammes dudit extrait de raisin.

5. Utilisation selon l'une des revendications 1 à 4, **caractérisée en ce que** ledit extrait contient de 10 à 60% en poids de proanthocyanidols (OPC).

6. Utilisation selon l'une des revendications 1 à 5, **caractérisée en ce que** ledit extrait contient de 0,001 à 0,1% en poids de trans-resvératrol.

7. Utilisation selon l'une des revendications 1 à 6, **caractérisée en ce que** ledit extrait est obtenu à partir de marc de raisin.

8. Utilisation selon l'une des revendications 1 à 6, **caractérisé en ce que** ledit extrait est obtenu à partir de pépins et/ou enveloppes de grains de raisin.

9. Procédé de traitement cosmétique du corps humain contre la cellulite et/ou la surcharge pondérale, **caractérisé en ce qu'**il implique l'administration par voie orale d'un extrait de raisin, riche ou enrichi en polyphénols, à raison de 30 à 90 % en poids de polyphénols.

10. Procédé de traitement cosmétique selon la revendication 9, **caractérisé en ce qu'**il implique l'administration d'une dose journalière de 0,2 à 2 grammes dudit extrait de raisin.

## Patentansprüche

1. Verwendung eines Weintrauben-Extraktes, der reich an oder angereichert mit Polyphenolen in einem Verhältnis von 30 bis 90 Gewichts-% Polyphenolen ist und zur Verabreichung auf oralem Weg bestimmt ist, um Cellulite und/oder Übergewicht zu behandeln.

2. Verwendung eines Weintrauben-Extraktes, der reich an oder angereichert mit Polyphenolen in einem Verhältnis von 30 bis 90 Gewichts-% an Polyphenolen ist, für die Herstellung eines Medikaments, das zur Verabreichung auf oralem Weg bestimmt ist, um Adipositas zu behandeln.

3. Verwendung nach Anspruch 1, **dadurch gekennzeichnet, dass** sie die Verabreichung einer täglichen Dosis von 0,2 bis 2 Gramm des Weintrauben-Extraktes beinhaltet.

4. Verwendung nach Anspruch 2, **dadurch gekennzeichnet, dass** das Medikament in Form einer Dosierungseinheit abgepackt ist, die für eine Dosis der täglichen Verwendung von 0,2 bis 2 Gramm des Weintrauben-Extraktes bestimmt ist.

5. Verwendung nach einem der Ansprüche 1 bis 4, **dadurch gekennzeichnet, dass** der Extrakt 10 bis 60 Gewichts-% Proanthocyanidole (OPC) enthält.

6. Verwendung nach einem der Ansprüche 1 bis 5, **dadurch gekennzeichnet, dass** der Extrakt 0,001 bis 0,1 Gewichts-% trans-Resveratrol enthält.

7. Verwendung nach einem der Ansprüche 1 bis 6, **dadurch gekennzeichnet, dass** der Extrakt ausgehend von Weintraubentrester erhalten wird.

8. Verwendung nach einem der Ansprüche 1 bis 6, **dadurch gekennzeichnet, dass** der Extrakt ausgehend von Kernen und/oder Beerenschalen von Weintrauben erhalten wird.

9. Verfahren zur kosmetischen Behandlung des menschlichen Körpers gegen Cellulite und/oder Übergewicht, **dadurch gekennzeichnet, dass** es die Verabreichung auf oralem Weg eines Weintrauben-Extraktes beinhaltet, der reich an oder angereichert mit Polyphenolen mit einem Verhältnis von 30 bis 90 Gewichts-% Polyphenolen ist.

10. Verfahren zur kosmetischen Behandlung nach Anspruch 9, **dadurch gekennzeichnet, dass** es die Verabreichung einer täglichen Dosis von 0,2 bis 2 Gramm des Weintrauben-Extraktes beinhaltet.

## Claims

1. Use of a grape extract which is rich or enriched in polyphenols, to a proportion of from 30% to 90% by weight of polyphenols, for oral administration, for treating cellulite and/or excess weight.

2. Use of a grape extract which is rich or enriched in polyphenols, to a proportion of from 30% to 90% by weight of polyphenols, for the manufacture of a medicament for oral administration, for treating obesity.

3. Use according to Claim 1, **characterized in that** it involves the administration of a daily dose of from 0.2 to 2 grams of said grape extract.

4. Use according to Claim 2, **characterized in that** the medicament is packaged in a unit dosage form intended for a daily usage dose of from 0.2 to 2 grams of said grape extract.

5. Use according to one of Claims 1 to 4, **characterized in that** said extract contains from 10% to 60% by weight of proanthocyanidols (PCOs).

6. Use according to one of Claims 1 to 5, **characterized in that** said extract contains from 0.001% to 0.1% by weight of trans-resveratrol.

7. Use according to one of Claims 1 to 6, **characterized in that** said extract is obtained from grape marc.

8. Use according to one of Claims 1 to 6, **characterized in that** said extract is obtained from grape pips and/or grape seed shells.

9. Cosmetic process for treating the human body against cellulite and/or excess weight, **characterized in that** it involves the oral administration of a grape extract, which is rich or enriched in polyphenols, to a proportion of from 30% to 90% by weight of polyphenols.

10. Cosmetic treatment process according to Claim 9, **characterized in that** it involves the administration of a daily dose of from 0.2 to 2 grams of said grape extract.
